# EUROPEAN PATENT APPLICATION

(11) **EP 2 104 157 A1**
(43) Date of publication of application: **23.09.2009**
(21) Application number: 07860204.2
(22) Date of filing: 26.12.2007
(51) Int. Cl.: H01L 51/50, C07C 15/27, C09K 11/06, H05B 33/10

(54) **SOLUTION CONTAINING ORGANIC EL MATERIAL, METHOD FOR SYNTHESIS OF ORGANIC EL MATERIAL, COMPOUND SYNTHESIZED BY THE SYNTHESIS METHOD, METHOD FOR FORMATION OF THIN FILM OF ORGANIC EL MATERIAL, THIN FILM OF ORGANIC EL MATERIAL, ORGANIC EL ELEMENT**

(30) Priority: 29.12.2006 JP 2006356888
(71) Applicant: Idemitsu Kosan Co., Ltd., Chiyoda-ku Tokyo 100-8321 (JP)
(72) Inventor: INOUE, Tetsuya, Sodegaura-shi Chiba 299-0293 (JP); ITO, Mitsunori, Sodegaura-shi Chiba 299-0293 (JP); KUBOTA, Mineyuki, Sodegaura-shi Chiba 299-0293 (JP)
(74) Representative: Gille Hrabal Struck Neidlein Prop Roos
(86) International application number: PCT/JP2007/074975
(87) International publication number: WO 2008/081823

(57) **Abstract**

An organic electroluminescent material-containing solution contains an organic electroluminescent material and a solvent. The organic electroluminescent material contains a host and a dopant. the host contains an organic electroluminescence-functional portion A formed of a low-molecular organic electroluminescent material and a soluble portion B bonded to the organic electroluminescence-functional portion A for solubilizing the low-molecular organic electroluminescent material in the solvent, the host being represented by a formula of A-B-E (where E represents a terminal group). The portion A is a low-molecular organic electroluminescent material having a central anthracene skeleton. The portion B is formed by bonding structures represented by the following formula (3) with structure(s) represented by the following formula (4). Units represented by the formula (3) include bonds in ortho positions or meta positions while unit(s) represented by the formula (4) includes bonds in para positions. When (a) represents the number of the structures represented by the formula (3) while (b) represents the number of the structure(s) represented by the formula (4), the number (a) and the number (b) satisfies both relationships respectively represented by 3 ≤ (a) + (b) ≤ 20 and (a) ≥ (b).

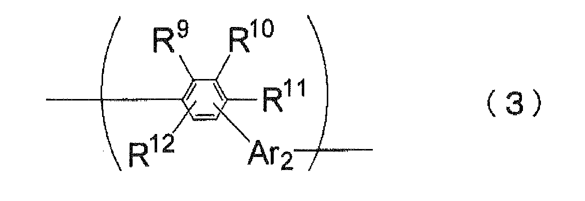

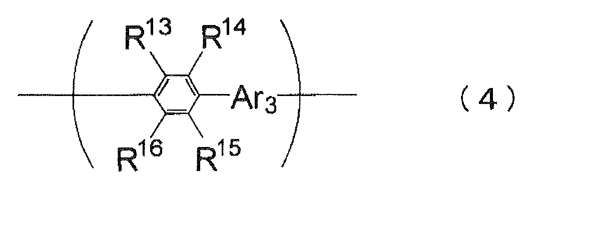

## Description

### TECHNICAL FIELD

The present invention relates to organic electroluminescent material-containing solution used for forming organic film(s) used for forming an organic electroluminescence device by a coating method, a method of synthesizing an organic electroluminescent material used in the solution, compounds synthesized by the synthesizing method, a method of forming thin film(s) of the organic electroluminescent material using the solution, thin film(s) of an organic electroluminescent material formed by the forming method, and an organic electroluminescence device.

### BACKGROUND ART

There has been known an organic electroluminescence device that utilizes luminescence of organic compound(s). The organic electroluminescence device includes a plurality of organic thin films laminated between an anode and a cathode. As organic-electroluminescent materials, a polymer material and a low-molecular material have been known. Since the low-molecular material is advantageous in terms of its simplified synthetic pathways and high degree of purification, development of low-molecular organic luminescent materials has been promoted. Some of the low-molecular organic electroluminescent materials have been reported to be favorably excellent in efficiency, a life duration and color purity and promoted to be put into practical use.
As a method of forming thin films from a low-molecular organic electroluminescent material, a vacuum deposition method has been adopted. According to the vacuum deposition method, a material is sublimated with favorable thermal stability to be deposited on a substrate, such that an organic electroluminescence device of high performance is provided (for instance, Patent Document 1).
However, high-vacuum facilities and complicated manufacturing processes are required for the vacuum deposition method.

As another method of forming films from an organic electroluminescent material, a coating method has been known. According to the coating method, which is generally used for forming films from a high-molecular organic electroluminescent material, an organic electroluminescent material dissolved in a solvent is used for forming thin films of the organic electroluminescent material (for instance, Patent Document 2). According to the coating method, thin films can be favorably formed from the organic electroluminescent material in a simplified manner.
In forming thin films from an organic electroluminescent material by the coating method, the organic electroluminescent material is required to be dissolved in a solvent. A composition prepared by dissolving a high-molecular organic electroluminescent material in a solvent is generally known to be used for the coating method.
Examples of the solvent include toluene, xylene, tetralin, mesitylen, cyclohexylbenzene and isopropyl biphenyl.

Patent Document 1: JP-A-2002-154993
Patent Document 2: JP-T-2004-536896

### DISCLOSURE OF THE INVENTION

### PROBLEMS TO BE SOLVED BY THE INVENTION

However, a low-molecular organic electroluminescent material, which is an insoluble material, is not favorably dissolved in such a solvent as described above in forming films from the low-molecular organic electroluminescent material.
While the coating material is not applicable to materials whose solubility is less than a predetermined value (e.g. 1 wt%), a low-molecular organic electroluminescent material generally exhibits solubility of 0.1 wt% to 0.2 wt%. Accordingly, such a low solubility of the low-molecular material has prevented the coating method from being applied to forming films from the low-molecular organic electroluminescent material.

In addition, even when the low-molecular organic electroluminescent material is dissolved in a solvent, viscosity may not be sufficient. In forming films by such a coating method, methods such as an ink-jet method and a nozzle-printing method are known to be used. Viscosity required for the methods is 1 cP or more.
When dissolved in a solvent, a high-molecular organic electroluminescent material can contribute to high viscosity of the solution. In contrast, since a low-molecular organic electroluminescent material does not exhibit high viscosity even when dissolved in a solvent, a thickener is required to be separately added so as to increase the viscosity.
An alcohol-based solvent, which is known as an example of such a thickener, is a poor solvent for the low-molecular organic electroluminescent material.
Addition of the poor solvent as the thickener as described above causes the solubility to become even lower.

The above-described problem(s) have prevented films from being formed from the low-molecular organic electroluminescent material (i.e., a material that is favorably excellent in light emission efficiency, a long life duration and color purity) by the coating method in a simplified manner at low cost, thereby awfully hampering a full-scale practical realization of the organic electroluminescent material.

An object of the present invention is to solve the above problem(s) and to provide an organic electroluminescent material-containing solution applicable to a coating method. Another object of the present invention is to provide a method for synthesizing an organic electroluminescent material, compound(s) synthesized by the method, a method of forming thin film(s) of an organic electroluminescent material, the thin film(s) of the organic electroluminescent material and an organic electroluminescence device.

### MEANS FOR SOLVING THE PROBLEMS

An organic luminescent material-containing solution according to an aspect of the present invention contains an organic electroluminescent material; and a solvent, in which
the organic electroluminescent material contains a host and a dopant,
the host is a compound containing: an organic electroluminescence-functional portion A formed of a low-molecular organic electroluminescent material; and a soluble portion B bonded to the organic electroluminescence-functional portion A for solubilizing the low-molecular organic electroluminescent material in the solvent, the compound being represented by following formula (1),
the organic electroluminescence-functional portion A in the formula (1) is represented by following formula (2),
the soluble portion B in the formula (1) is formed by bonding either plural structures represented by following formula (3) or plural structures represented by following formula (4) or by bonding plural structures represented by the formula (3) with structure(s) represented by the formula (4), and
a number (a) and a number (b) satisfies both relationships respectively represented by 3 ≤ (a) + (b) < 20 and (a) ≥ (b), the number (a) being the number of the structures represented by the formula (3), the number (b) being the number of the structure(s) represented by the formula (4).

[Formula 1] A-B-E (1)

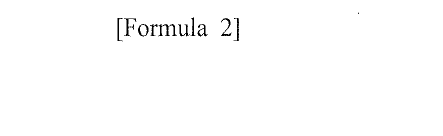

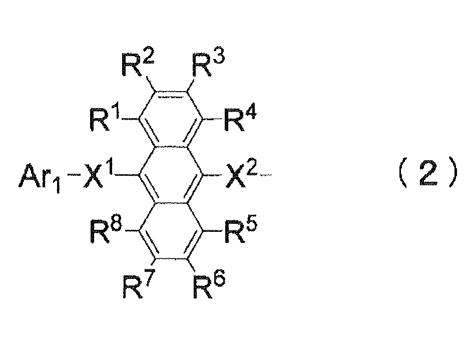

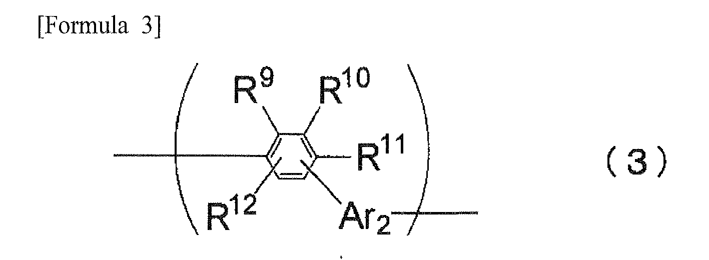

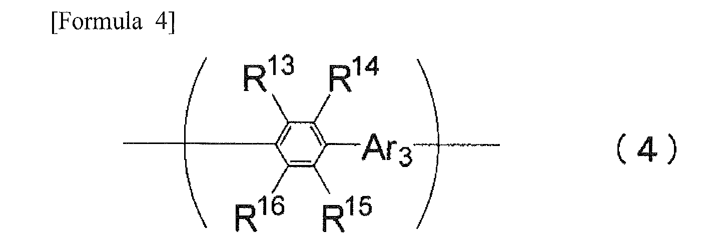

In the formulae: Ar₁, Ar₂ and Ar₃ each represent a substituted or unsubstituted aromatic hydrocarbon group having 6 to 50 carbon atoms;
X¹ and X² each represent a single bond or a divalent substituted or unsubstituted aromatic hydrocarbon group having 6 to 50 carbon atoms; and
R¹ to R¹⁶ each are selected from a group consisting of a hydrogen atom, a substituted or unsubstituted aromatic hydrocarbon group, a substituted or unsubstituted aromatic heterocyclic group, a substituted or unsubstituted alkyl group, a substituted or unsubstituted cycloalkyl group, a substituted or unsubstituted alkoxy group, a substituted or unsubstituted aralkyl group, a substituted or unsubstituted aryloxy group, a substituted or unsubstituted arylthio group, a substituted or unsubstituted alkoxycarbonyl group, a substituted or unsubstituted silyl group, a carboxyl group, a halogen atom, a cyano group, a nitro group and a hydroxyl group. An adjacent set of R¹ to R¹⁶ is allowed to be mutually bonded to form a cyclic structure.

In the solution prepared as described above, although the low-molecular organic electroluminescent material itself does not exhibit favorable solubility in the solvent, the structure B in the above formula (1) contained in the low-molecular organic electroluminescent material as the soluble portion can increase the solubility in the solvent.
In order to merely solubilize the material in the solvent, a possible method of solubilizing the material may be to add, for instance, a polar group as a substituent to a central anthracene skeleton.
However, when the central anthracene skeleton of the organic electroluminescence-functional portion is added with a polar substituent as described above, function(s) as the organic electroluminescent material may be impaired, an exemplarily consequence of which may be that luminescent performance is drastically deteriorated or that its life is notably shortened.
Additionally, another possible method of solubilizing the material may be to polymerize the material by adding, for instance, a molecular chain having a molecular weight of 10,000 or more to the organic electroluminescence-functional portion.
However, the addition of such a large molecular chain to the organic electroluminescence-functional portion A as mentioned above may prevent the organic electroluminescence-functional portion from sufficiently functioning and hamper sufficient functionality thereof.
According to the aspect of the present invention, the soluble portion B is provided at such a position as not to impair the electroluminescent performance of the functional portion A.

Further, the soluble portion B is formed by bonding structures represented by the formula (3) and structure(s) represented by the formula (4) together such that an upper limit to a number U (= (a) + (b)) obtained by adding the number of the structures represented by the formula (3) with that of the structure(s) represented by the formula (4) is 20.
By restricting the size of the soluble portion and making the material serve as an oligomer as described above, the organic electroluminescence-functional portion can sufficiently function and exhibit sufficient solubility in the solvent.
Particularly, unlike a dopant for which even low solubility is sufficient, a host is required to exhibit solubility that is equal to or more than a predetermined value for forming a sufficiently thick film by the coating method. According to the present invention, the organic electroluminescence-functional portion represented by A of the above formula (1) can be solubilized, thereby enabling the film(s) to be formed by the coating method.
A lower limit to the value of (a) + (b) is set at 3 so that the soluble portion B can properly function.

Although the organic electroluminescence-functional portion A can be solubilized by the addition of the soluble portion B, experiment(s) have proved that the solubility is not enhanced when the molecular chain of the soluble portion B has excessively many bonds in para positions.
Hence, in the present invention, the formula (3) represents a structure in which at least one bond is situated in an ortho position or a meta position, the formula (4) represents a structure in which at least one bond is situated in a para position, and the number of the structures represented by the formula (3) is equal to or larger than the number of the structure(s) represented by the formula (4) (i.e., (a) ≥ (b)).
By making the number (a) of the structures represented by the formula (3) equal to or larger than the number (b) of the structure(s) represented by the formula (4), the number of such structures having bonds in para positions as represented by the formula (4) can be restricted, thereby enhancing the function(s) of the soluble portion B to enhance the solubility.
Solubilized by the short soluble portion B, the low-molecular organic electroluminescent material can be solubilized without impairing the function(s) of the organic electroluminescence-functional portion A.

In addition, an organic electroluminescent material-containing solution is required to have a viscosity of a predetermined value or more. A conventional problem has been that the solution cannot have a sufficient viscosity by merely dissolving the low-molecular organic luminescent material in the solvent, so that the solution has been unsuitable for the coating method.
According to the aspect of the present invention, since the solubility of the organic electroluminescent material is enhanced, solubility required for forming the film(s) by the coating method can be sufficiently secured even when a viscosity control reagent is mixed into the solvent.
For instance, even when a poor solvent such as an alcohol-based solvent is used as the viscosity control reagent, the solubility of the organic electroluminescent material can be sufficiently secured, so that the organic electroluminescent material-containing solution that is excellent in solubility and viscosity and applicable to forming the films by the coating method can be obtained.
Further, according to the present invention, since the low-molecular organic electroluminescent material is added with a molecular chain, viscosity of the solution in which the material is dissolved can be increased.
Even when an alcohol-based solution needs to be added as a thickener, the additive amount of the thickener can be reduced.

Such a compound having a central anthracene skeleton is suitable for a host of a blue emitting layer. According to the aspect of the present invention, the host suitable for the blue emitting can be solubilized, so that the blue emitting layer can be formed by the coating method.
Conventionally, although films of emitting layers can be formed by the coating method when a polymer-based material is used, no polymer-based material suitable for the high-performance blue emitting layer has been found. According to the aspect of the present invention, a host material for a higher-performance blue emitting layer can be solubilized to be applicable to the coating method, an effect of which is highly valuable.

Although an example of E situated at a terminal of the formula (1) is H (hydrogen atom), the E is not limited thereto.

Now, a host and a dopant will be described below.
An organic electroluminescence device is provided by laminating such functional layers as a hole injecting layer, a hole transporting layer, an emitting layer, an electron transporting layer and an electron injecting layer.
In the emitting layer formed by a host and a dopant, phenomena such as energy transfer arises from the host to the dopant, so that the dopant emits light.
The dopant is added (doped) to the host with an exemplarily ratio of the dopant to the host being 0.01 to 20 wt%.
Since the host occupies a major portion (e.g. 80 % or more) of the emitting layer of 30 nm to 100 nm, the host is required be dissolved in the organic electroluminescent material-containing solution by a predetermined amount for forming a film of the emitting layer through coating processes.
According to the present invention, the organic electroluminescent material-containing solution that is suitable for forming a film by coating is provided.

According to the aspect of the present invention, it is preferable that the structures represented by the formula (4) are not located next to each other in a structure of the soluble portion B of the formula (1).

The solubility is deteriorated when a large number of the structures represented by the formula (4) are included in the soluble portion B of the formula (1), particularly when bonds in para positions are consecutively included as in the above formula (4).
According to the present invention, since the structures represented by the formula (4) are not consecutively included, the soluble portion B can enhance the solubility.
In addition, even a short soluble portion can enhance the solubility, such that the function(s) of the organic electroluminescence-functional portion can be sufficiently retained.

According to the aspect of the present invention, it is preferable that the Ar₂ in the formula (3) and the Ar₃ in the formula (4) in the soluble portion B are respectively a phenylgroup.

By adopting phenyl groups for Ar₂ and Ar₃ in the structures represented by the formulae (3) and (4), the soluble portion B becomes easily bendable, thereby enhancing the solubility in the solvent.
With this arrangement, even when a length of the soluble portion B in the formula (1) is shortened, required solubility can be sustained.
Hence, a host material with high solubility, in which the length of the soluble portion B is shortened while the function(s) of the organic electroluminescence-functional portion A is sufficiently sustained, is provided.

According to the aspect of the present invention, it is preferable that the soluble portion is formed by bonding the structures represented by the formula (3) with the structure(s) represented by the formula (4), and that a dispersion of a bonding number U is a single value, the bonding number U representing a sum of the number (a) of the structures represented by the formula (3) and the number (b) of the structure(s) represented by the formula (4).

By uniforming the molecular weight or the size of the host material with the dispersion of the bonding number U (= (a) + (b)) being set at a single value, functions as the organic electroluminescent material can be stabilized.
Values of the polymerization degree are dispersed in a predetermined range in a general polymer or oligomer. Accordingly, when a size differs from a molecular to a molecular, functions as an organic electroluminescent material are not stabilized, such that performance of a predetermined level may not be secured for an organic electroluminescent device formed of the organic electroluminescent material.
According to the present invention, since the sizes of the molecular are uniformed by narrowing a dispersion range of the bonding number U, the performance can be stabilized.
Although it is generally not possible to narrow the dispersion of the polymerization degree by controlling the polymerization degree when the polymerization degree is large as in a polymer, the bonding number can be controlled during a synthesizing process because the bonding number U (= (a) + (b)) is set at 20 or less according to the present invention.

"Setting the bonding number U (= (a) + (b)) at a single value" means that the bonding number has a peak of a single mode when the dispersion thereof is measured. For instance, when a reaction is conducted with a target bonding number U (= (a) + (b)) being 5, the bonding number U is preferably 5 throughout approximately 60% or more of the host.
In addition, when the reaction is conducted with the target bonding number U being 5, the bonding number U is more preferably 5 throughout approximately 70% or more of the host, further preferably 5 throughout approximately 90% or more of the host and the most preferably 5 throughout all (100%) of the host.

Note that the dispersion measured may widely range. For instance, although the molecular weight is required to be uniform when films are formed by a vapor deposition method, a dispersion of the molecular weight does not affect film forming processes when films are formed by the coating method.
When the bonding number U may be dispersed, synthesizing requirements can be relaxed and synthesizing processes can be simplified.

According to the aspect of the present invention, it is preferable that the soluble portion B is entirely formed of the structures represented by the formula (3).

When such a structure is adopted, the organic electroluminescent material can exhibit the highest solubility.

According to the aspect of the present invention, it is preferable that the solvent is selected from a group consisting of an aromatic solvent, a halogen-based solvent and an ether-based solvent.

As described above, by selecting the solvent from the group consisting of aromatic solvent, halogen-based solvent and ether-based solvent, the organic electroluminescent material according to the present invention can be dissolved in the solvent by a required amount or more (e.g. 1 wt%).
By adding such a viscosity control reagent as is selected from the group consisting of alcohol-based solution, ketone-based solution, paraffin-based solution and alkyl substituted aromatic solution with carbon atoms of 4 or more, the viscosity of the organic electroluminescent material-containing solution can be increased so as to be suitable for several types of coating methods (the ink-jet method, the nozzle-printing method and a spin coat method).
The solvent may be at least one solvent selected from the group consisting of aromatic solvent, halogen-based solvent and ether-based solvent, and the solvent may be prepared by mixing two or more solvents selected therefrom.
Likewise, the viscosity control reagent may be at least one solution selected from the group consisting of alcohol-based solution, ketone-based solution, paraffin-based solution and alkyl substituted aromatic solution with carbon atoms of 4 or more, and the agent may be prepared by mixing two or more solutions selected therefrom.

Since the host is solubilized by the addition of the molecular chain in the present invention, the viscosity of the solution is increased by an amount of the molecular chain when the host is dissolved in the solvent.
Accordingly, even when the viscosity control reagent is added for controlling the viscosity, the additive amount of the viscosity control reagent can be reduced.

According to the aspect of the present invention, it is preferable that the solvent is selected from a group consisting of an aromatic solvent, a halogen-based solvent and an ether-based solvent, and that the solvent is further added with a viscosity control reagent selected from a group consisting of an alcohol-based solution, a ketone-based solution, a paraffin-based solution and an alkyl-substituted aromatic solution having 4 or more carbon atoms.

When an alcohol-based solution is used as the viscosity control reagent, cares must be paid to the conservation management of the agent because the alcohol-based solution easily absorbs moisture. In contrast, when an alkyl substituted aromatic solution with carbon atoms of 4 or more is used as the viscosity control reagent, the conservation management of the agent can be advantageously simplified because the solution is hydrophobic.
By changing a structure(s) of an alkyl group(s) (e.g. lengthening the alkyl chain) in the alkyl substituted aromatic solution with carbon atoms of 4 or more, the viscosity control reagent can advantageously perform a viscosity control.
On the other hand, the alcohol-based solution, which is highly viscous, is preferable in preparing a solution that is suitable for a film forming process requiring high solution viscosity (e.g., inkjet printing).

A type or an additive amount of the viscosity control reagent can be properly selected in accordance with the viscosity required for various types of film forming processes.
The "alkyl substituted aromatic solution with carbon atoms of 4 or more" means an aromatic having alkyl substituents with carbon atoms of 4 or more.
Although there is no specific upper limit for carbon atoms of the alkyl substituents, the upper limit may be exemplarily set around 50.

According to the aspect of the present invention, it is preferable that the solvent is the aromatic solvent, and that the viscosity control reagent is the alcohol-based solution or the alkyl-substituted aromatic solution having 4 or more carbon atoms.

When the alcohol-based solution is used as the viscosity control reagent, cares must be paid to the conservation management of the agent because the alcohol-based solution easily absorbs moisture. In contrast, when the alkyl substituted aromatic solution with carbon atoms of 4 or more is used as the viscosity control reagent, the conservation management of the agent can be advantageously simplified because the solution is hydrophobic.
By changing a structure(s) of an alkyl group(s) (e.g. lengthening the alkyl chain) in the alkyl substituted aromatic solution with carbon atoms of 4 or more, the viscosity control reagent can advantageously perform a viscosity control.
On the other hand, the alcohol-based solution, which is highly viscous, is preferable in preparing a solution that is suitable for a film forming process requiring high solution viscosity (e.g., ink jet printing).

A type or an additive amount of the viscosity control reagent can be properly selected in accordance with the viscosity required for various types of film forming processes.
The "alkyl substituted aromatic solution with carbon atoms of 4 or more" means an aromatic having alkyl substituents with carbon atoms of 4 or more.
Although there is no specific upper limit for carbon atoms of the alkyl substituents, the upper limit may be exemplarily set around 50.

A method of synthesizing the organic electroluminescent material according to another aspect of the present invention includes: synthesizing the low-molecular organic luminescent material as the organic electroluminescence-functional portion A; synthesizing a molecular chain as the soluble portion B; and bonding the low-molecular organic electroluminescent material synthesized in the step of synthesizing the low-molecular organic electroluminescent material with the molecular chain synthesized in the step of synthesizing the molecular chain.

A compound according to still further aspect of the present invention is synthesized by the above-described synthesizing method.

In the synthesizing method described above, the organic electroluminescent material is synthesized in the step for synthesizing the low-molecular organic electroluminescent material, the molecular chain is synthesized in the step for synthesizing the molecular chain, and the organic electroluminescent material that is to be dissolved in the organic electroluminescent material-containing solution is synthesized by bonding the low-molecular organic electroluminescent material and the molecular chain.
Accordingly, by synthesizing the highly-purified low-molecular organic electroluminescent material and the molecular chain whose bonding number U has been controlled to be at a predetermined value, the highly-purified organic luminescent material with the bonding number being controlled to be constant can be obtained.
When the organic electroluminescent material that is highly soluble in the solvent is synthesized through a single step, such a purification method as a reprecipitation method and a recrystallization method may not be adopted because of the high solubility of the material. Thus, a column purification method, for instance, is a possible option. However, the column purification method is not practical in that the method requires huge amount of time for purifying the material of several kilograms.
According to the present invention, the low-molecular organic electroluminescent material having been highly purified is bonded with the molecular chain used for oligomerization, thereby providing the highly-purified organic electroluminescent material of excellent performance with solubility in the solvent.

A method of forming thin films of the organic electroluminescent material according to still further aspect of the present invention includes: dropping the above-described organic electroluminescent material-containing solution on a formation area; and forming film(s) of the organic electroluminescent material by evaporating the solvent in the organic-electroluminescence-material-containing solution dropped in the step of dropping.

A thin film of the organic electroluminescent material according to still further aspect of the present invention is formed by the above-described method of forming thin film(s) of an organic electroluminescent material.

An organic electroluminescent device according to still further aspect of the present invention includes the above-described thin film(s) of an organic electroluminescent material.

According to the aspect of the present invention, it is preferable that the organic electroluminescence device further includes an emitting layer that comprises the thin film(s) formed from the organic electroluminescent material-containing solution by coating method, in which the emitting layer emits light of blue color.

According to such an arrangement, the organic electroluminescence device of blue light-emitting can be obtained by the coating method.
A polymer has been known to be applicable to an organic electroluminescent material to be used in the coating method.
However, although some of such polymers have been known to be applicable to a red emitting material and a green emitting material, none of such polymers has been known to be applicable to a blue emitting material.
For this reason, it has not been possible to form the blue emitting layer by the coating method.
The host material according to the aspect of the present invention is suitable for the host material of the blue emitting layer, thereby enabling the films of the blue emitting layer to be formed by the coating method.

### BEST MODE FOR CARRYING OUT THE INVENTION

Embodiments according to the present invention will be described.

An organic electroluminescent material-containing solution according to an aspect of the present invention is prepared by dissolving an organic electroluminescent material in a solvent.
The organic electroluminescent material-containing solution contains a host and a dopant.

The host is represented by the following general formula:

A- B - E (1)

(E represents a terminal group)

A represents an organic electroluminescence-functional portion.
B represents a soluble portion.

As the organic electroluminescence-functional portion represented by A in the general formula, a material known as a low-molecular organic electroluminescent material whose central skeleton is anthracene can be preferably used.
The organic electroluminescence-functional portion is further preferably an organic electroluminescent material of an asymmetric anthracene derivative whose central skeleton is anthracene with substituents in ninth and tenth positions being asymmetric with respect to each other.
Specifically, the organic electroluminescence-functional portion A is represented by the following general formula (2):

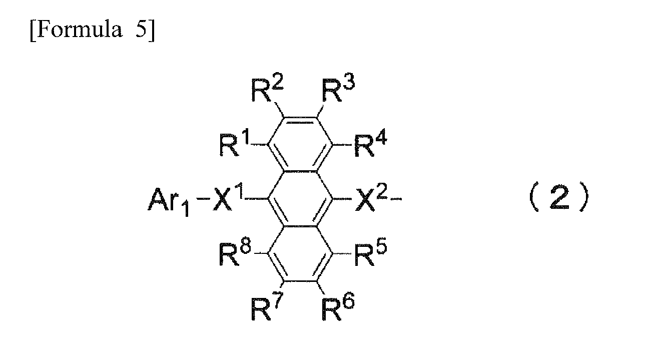

B serving as the soluble portion in the general formula (1) is a molecular chain formed by bonding basic units.
The number of the bonded units is 20 or less, and the molecular chain is what is called an oligomer.
The molecular chain is represented by the following formulae:
Specifically, the soluble portion B is a molecular chain formed by bonding either one of plural structures each represented by the formula (3) and structure(s) each represented by the formula (4), or bonding the plural structures represented by the formula (3) with the structure(s) represented by the formula (4).

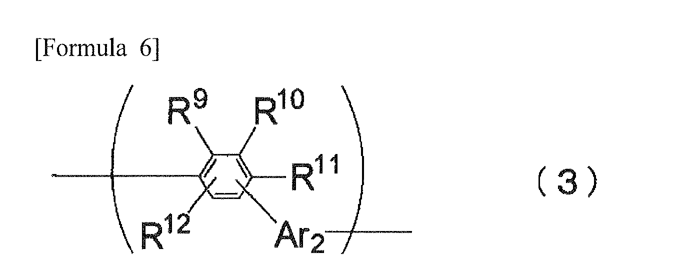

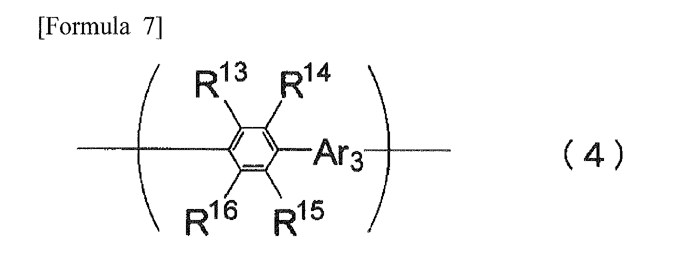

In the formulae: Ar₁, Ar₂ and Ar₃ each represent a substituted or unsubstituted aromatic hydrocarbon group having 6 to 50 carbon atoms; X¹ and X² each represent a single bond or a divalent substituted or unsubstituted aromatic hydrocarbon group having 6 to 50 carbon atoms; and R¹ to R¹⁶ each are selected from a group consisting of a hydrogen atom, a substituted or unsubstituted aromatic hydrocarbon group, a substituted or unsubstituted aromatic heterocyclic group, a substituted or unsubstituted alkyl group, a substituted or unsubstituted cycloalkyl group, a substituted or unsubstituted alkoxy group, a substituted or unsubstituted aralkyl group, a substituted or unsubstituted aryloxy group, a substituted or unsubstituted arylthio group, a substituted or unsubstituted alkoxycarbonyl group, a substituted or unsubstituted silyl group, a carboxyl group, a halogen atom, a cyano group, a nitro group and a hydroxyl group.
An adjacent set of R¹ to R¹⁶ may be mutually bonded to form a cyclic structure.

In forming the soluble portion B, a bonding order of the structures represented by the formula (3) and the structure(s) represented by the formula (4) does not subject to any restrictions.
For instance, the structures represented by the formulae (3) and (4) may be alternately bonded together to form an exemplary chain of (3) - (4) - (3) - (4) - (3) - (4). Alternatively, unit portions represented by the formula (3) or unit portions represented by the formula (4) may be consecutively bonded together to form an exemplary chain of (3) - (3) - (4) - (4) - (3) - (3).

It should be noted that the number (a) of the structures represented by the formula (3) is required to be equal to or larger than the number (b) of the structure(s) represented by the formula (4) for solubilization when the number of the structures represented by the formula (3) and that of the structure(s) represented by the formula (4) contained in the soluble portion B are compared.
In other words, the number (a) and the number (b) should be on the relationship of (a) ≥ (b). (in the inequality, (a) represents the number of the structures represented by the formula (3) while (b) represents the number of the structure(s) represented the formula (4).)
For further solubilization, the relationship is preferably (a) > (b) because the structure(s) represented by the formula (3) contributes to the solubilization more than the structure(s) represented by the formula (4).

In addition, for further solubilization, the structures represented by the formula (4) preferably do not neighbor each other in the soluble portion B.
For instance, when two structures represented by the formula (3) and two structures represented by the formula (4) are used, a chain of (3) - (4) - (3) - (4) is preferable over a chain of (3) - (3) - (4) - (4) because a consecution of the structures represented by the formula (4) leads to a consecution of the bonds in para positions and deteriorates the solubility.

More preferably, the entirety of the soluble portion (3) is formed by solely bonding the structures represented by the formulae (3).

While Ar₂ and Ar₃ in the formulae (3) and (4) do not subject to any specific limitations but may be selected as necessary, Ar₂ and Ar₃ are preferably, for instance, phenyl groups in order to enhance the solubility.
By adopting phenyl groups for Ar₂ and Ar₃ in the formulae (3) and (4), the soluble portion B becomes easily bendable to have a greater affinity for the solvent, thereby enhancing the solubility.

In other words, the entirety of the soluble portion B is preferably formed solely from the structures represented the formula (3), in which Ar₂ is preferably a phenyl group.
More preferably, the bonds with Ar₂ are all in ortho positions or meta positions.
Specifically, the following compound can be exemplified.

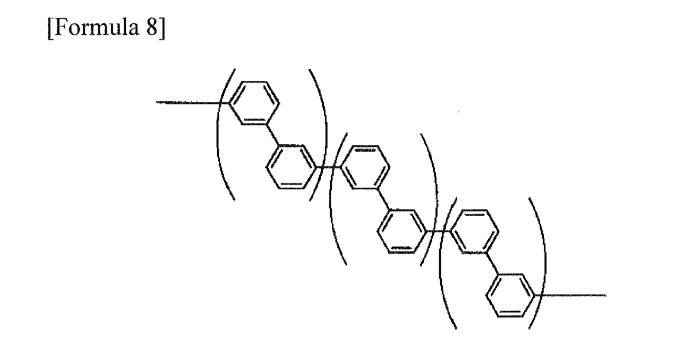

However, bonds with Ar₂ and Ar₃ should not be restrictively understood. Specifically, when Ar₂ and Ar₃ have benzene ring structures, bonds with the benzene rings may be in any one of ortho positions, meta positions or para positions.
Solubilization (i.e., an object of the present invention) can be realized when the number of the structures represented by the formula (3) having at least one bond in an ortho position or a meta position is equal to or larger than the number of the structure(s) represented by the formula (4) having at least one bond in a para position.

The length of the soluble portion B subjects to an upper limit and a lower limit.
Specifically, when the number of the structures represented by the formula (3) and the number of the structures represented by the formula (4) included in the soluble portion B are respectively represented by (a) and (b), the bonding number U (= (a) + (b)) obtained by adding the number of the structures represented by the formulae (3) with that of the structure(s) represented by the formula (4) is in a range of 3 or more to 20 or less.
When the bonding number U serving as the length of the soluble portion B is less than 3, required solubility may not be obtained. On the other hand, when the bonding number U exceeds 20, the size of the soluble portion B becomes so large that the organic electroluminescence-functional portion A may not sufficiently function.

In the soluble portion B formed by bonding the basic units represented by the formulae (3) and (4), the bonding number U is preferably a single value.
Specifically, although values of the polymerization degree in a general oligomer are dispersed in a predetermined range, the values of the bonding number in the present invention are preferably the same, so that molecular sizes are uniform.
By uniforming the molecular sizes, performance as the organic electroluminescent material can be stabilized.

Next, a synthesizing method will be described below.
In order to synthesize the host, the low-molecular organic electroluminescent material (A in the general formula) is initially synthesized.
Such a synthesizing method has been conventionally known.
Since the low-molecular organic electroluminescent material is insoluble, such preparation methods as reprecipitation, recrystallization and the like may be adopted. In addition, the low-molecular organic electroluminescent material may be prepared by sublimation.
A highly-purified organic electroluminescent material is prepared by the above methods.
Then, the molecular chain as the soluble portion (B in the general formula) is prepared.
The molecular chain is synthesized by polymerizing or sequentially synthesizing the above basic units (represented by the formulae (3) and (4)).
At this time, the bonding number U is controlled to be the same throughout all the molecular chain.
Generally, polymerization is to form a molecular chain by a single reaction of basic units of a predetermined amount dissolved in a solvent while sequential synthesis is to sequentially lengthen a molecular chain by a plurality of reactions of basic units so that each basic unit are sequentially bonded.
Subsequently, a host material is synthesized by bonding the low-molecular organic electroluminescent material with the molecular chain.

Examples of the host are shown below.

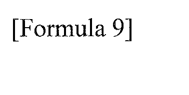

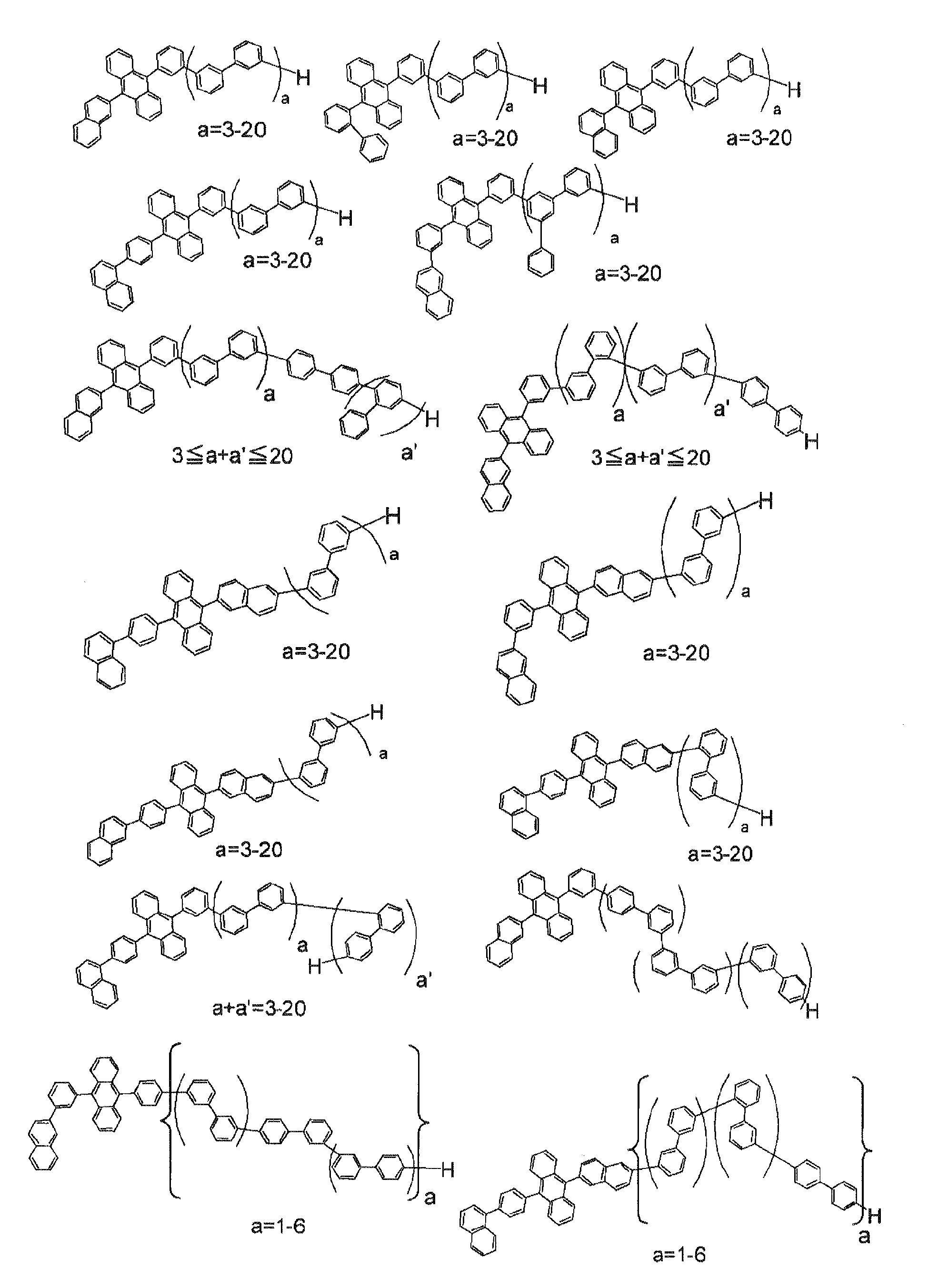

### (Synthesis Examples)

Examples of synthesized hosts are shown below.

### (Synthesis Example 1) Synthesis of Anthracene Compound (AN-1)

Under argon atmosphere, 3.3g of an intermediate body I₁ obtained by a known method and 2.6g of 3-(9-(naphthalene-2-yl) anthracene-10-yl) phenylboronic acid were dispersed in a solvent in which 80 milliliters of DME and 80 milliliters of toluene were mixed. The mixture was added with 0.20g of tetrakis(triphenylphosphine)palladium and 9ml of 2M-sodium carbonate aqueous solution and refluxed for eight hours.
After being left for a night, the mixture experienced filtration of precipitated crystal, washing by water and methanol and washing by heated toluene, so that 3.5g of the target compound (AN-1) was obtained in a form of a slightly yellow solid (yield: 69%).
When FD-MS (Field Desorption Mass Spectrometry) of the obtained compound was measured, m/z: 837 (calculated for C₆₆H₄₄: 837) was detected, whereby the obtained compound was identified to be AN-1.

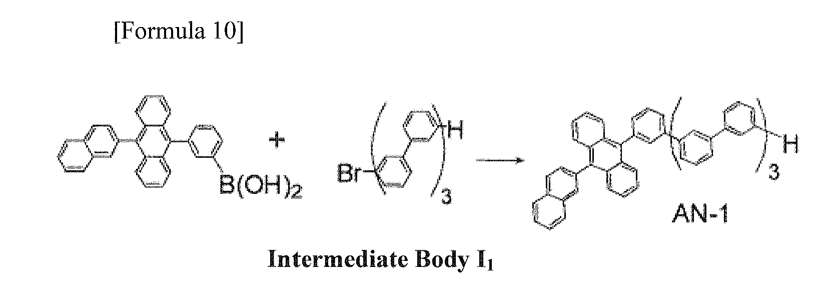

### (Synthesis Example 2) Synthesis of Anthracene Compound (AN-2)

In synthesizing AN-1 as described above, 6.1g of an intermediate body I₂ was used in place of the intermediate body I₁.
3.9g of the target compound (AN-2) was obtained in a form of a slightly yellow solid (yield: 50%).
When FD-MS (Field Desorption Mass Spectrometry) of the obtained compound was measured, m/z: 1293 (calculated for C₁₀₂H₆₈: 1293) was detected, whereby the obtained compound was identified to be AN-2.

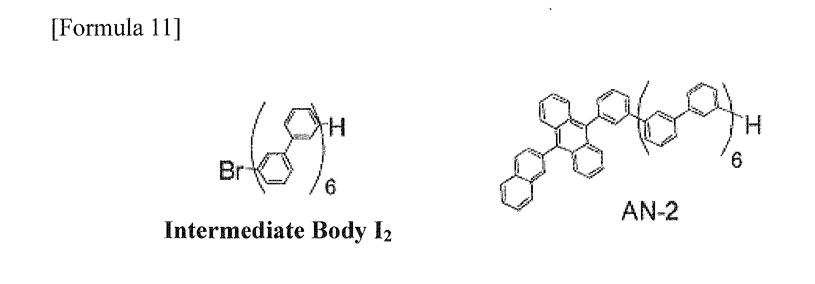

The solvent and a viscosity control reagent will be described below.
Examples of the solvent are an aromatic solvent with or without alkoxyl groups or halogen such as benzene, toluene, xylene, ethylbenzene, di-ethylbenzene, anisole, chlorobenzene, di-chlorobenzene, chlorotoluene and the like.
In addition, the solvent may be a halogenated hydrocarbon-based solvent such as dichloromethane, dichloroethane, chloroform, carbon tetrachloride, tetrachloroethane, trichloroethane and the like.
Further, the solvent may be an ether-based solvent such as dibutyl ether, tetrahydrofuran, dioxane and the like.

Examples of the viscosity control reagent are a linear or branched alcohol-based solvent such as methanol, ethanol, propanol, butanol, pentanol, hexanol, octanol, nonanol, cyclohexanol, methyl cellosolve, ethyl cellosolve, ethylene glycol, benzyl alcohol and the like.
Additionally, the viscosity control liquid may be an alkyl substituted aromatic solvent having carbon atoms of 4 or more with or without linear or branched alkyl groups such as butylbenzene, cyclohexylbenzene, tetralin, butylbenzene, dodecylbenzene and the like.

As the solvent or the viscosity control reagent, one of the above examples may be singularly used, or a mixture of plurality thereof may be used.

### (Solubility Evaluation)

Next, solubility evaluation examples will be described.

### (Solubility Evaluation 1)

In order to evaluate a relationship between the soluble portion B and the solubility, how the solubility was changed depending on the bonding number U of the solubility portion B was examined.
As the compound, an anthracene compound as represented by the following formula was used.
A solubility portion B of the compound was structured as represented by the formula (3), and the bonding number of the solubility portion is represented by (a).
With the above structure, compounds respectively having bonding numbers (a) of 1, 2, 3 and 6 were synthesized.
Then, 0.01g of the anthracene compound and 1g of toluene were put into a glass bottle to be stirred.
Whether or not any insoluble matter was present in the solution was observed by a visual check. Results of the evaluation are shown in the table below.

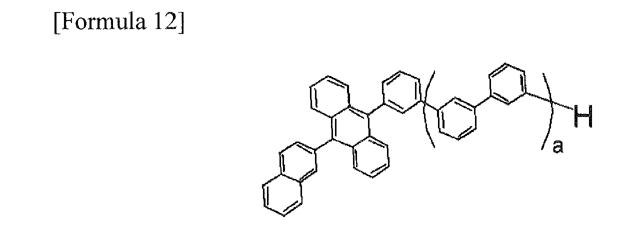

**[Table 1]**

| | (a) = 1 | (a) = 2 | (a) = 3 | (a) = 6 |
|---|---|---|---|---|
| | | | (AN-1) | (AN-2) |
| Presence of insoluble matter | Present | Present | Not Present | Not Present |

According to the above results, the compounds whose soluble portions B had bonding numbers of 2 or less exhibited solubility of 1 wt% or less.
In contrast, the compounds whose soluble portions B had bonding numbers of 3 or more exhibited solubility of 1 wt% or more.
In other words, it has been found that the bonding number U of the soluble portion B is required to be 3 or more in order for the host material applied to the coating method to exhibit sufficient solubility.

### (Solubility Evaluation 2)

In order to evaluate a relationship between unit(s) structuring the soluble portion B and the solubility, how the solubility was changed depending on the structure of the solubility portion B was examined.
Compounds used in the examination were AN-1, AN-3, AN-4 and an-1.
The compounds were different from one another in bonding positions of bonding groups in the solubility portions B.
The entirety of the soluble portion B of AN-1 was formed of basic units represented by the formula (3), in which the bonding positions of the bonding groups were all meta positions.
A soluble portion B of AN-3 was formed to be a chain of (3) - (4) - (3), in which the bonding positions of the bonding groups were a meta position, a para position and meta position.
A soluble portion B of AN-4 was formed to be a chain of (3) - (3) - (4), in which the bonding positions of the bonding groups were a meta position, a meta position and para position.
A soluble portion B of an-1 was formed to be a chain of (3) - (4) - (4), in which the bonding positions of the bonding groups were a meta position, a para position and para position.
For evaluation of the solubility, as in the solubility evaluation 1, 0.01g of the anthracene compound and 1g of toluene were put into a glass bottle to be stirred. Then, whether or not any insoluble matter was present in the solution was observed by a visual check.

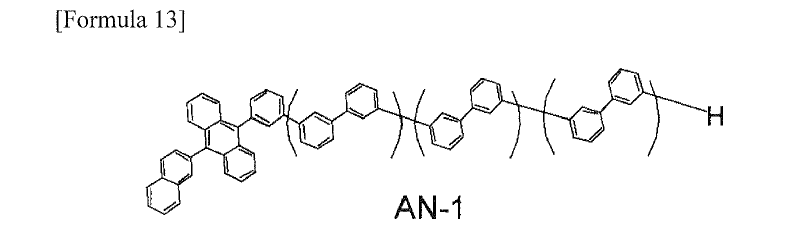

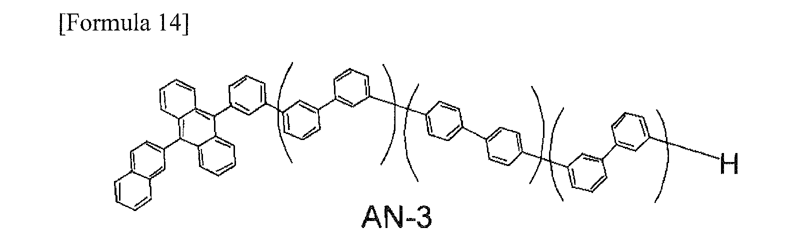

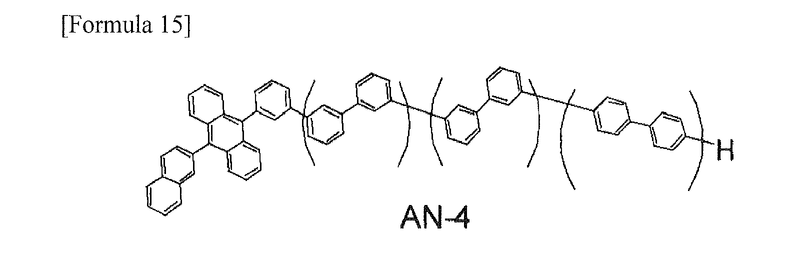

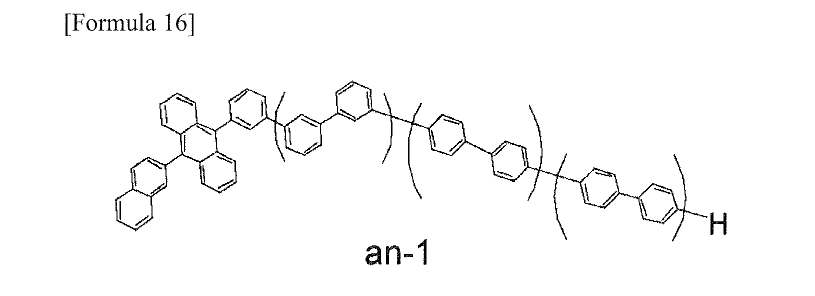

The evaluation result of the solubility of AN-1, in which the bonding positions were all meta positions, was that there was no insoluble matter present therein, and that the solubility in toluene was 1 wt% or more.
The evaluation result of the solubility of AN-3, in which the bonding positions were the meta position, the para position and the meta position, was that there was no insoluble matter present therein, and that the solubility in toluene was 1 wt% or more.
The evaluation result of the solubility of AN-4, in which the bonding positions were the meta position, the meta position and the para position, was that there was no insoluble matter present therein, and that the solubility in toluene was 1 wt% or more.
The evaluation result of the solubility of an-1, in which the bonding positions were the meta position, the para position and the para position, was that there was insoluble matter present therein, and that the solubility in toluene was 1 wt% or less.
It has been found that, although the structure represented by the formula (4) where the bonding groups necessarily include bonds in para positions may be present in the soluble portion, a consecution of the structures represented by the formula (4) as in the chain of (4) - (4) of an-1 considerably deteriorates the solubility.

### (Dopant)

Next, the dopant will be described below. Examples of the dopant are a styryl amine compound and/or an arylamine compound. An example of the styryl amine compound is represented by the following general formula (A) while an example of the arylamine compound is represented by the following formula (B).

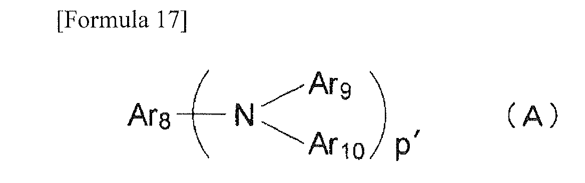

In the general formula (A), at least one of Ar₈ to Ar₁₀ includes a styryl group.
Ar₈ is selected from a group consisting of a phenyl group, a biphenyl group, a terphenyl group, a stilbene group, a distyryl-aryl group while Ar₉ and Ar₁₀ are substituted or unsubstituted aromatic groups having hydrogen atoms or carbon atoms of 6 to 20. p' is an integer in a range of 1 to 4.

The aromatic group having carbon atoms of 6 to 20 is preferably a phenyl group, a naphthyl group, an anthracenyl group, a phenanthryl group, a terphenyl group or the like.

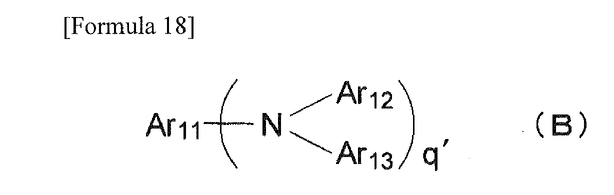

In the general formula (B), Ar₁₁ to Ar₁₃ are substituted or unsubstituted aryl groups having the number of carbon atoms forming the aromatic ring of 5 to 40. q' is an integer in a range of 1 to 4.

The aryl groups having the number of atoms forming a ring of 5 to 40 are preferably phenyl groups, naphthyl groups, anthracenyl groups, phenanthryl groups, crycenyl groups, pyrenyl groups, coronyl groups, biphenyl groups, terphenyl groups, pyrroryl groups, furanyl groups, thiophenyl groups, benzothiophenyl groups, oxadiazolyl groups, diphenylanthracenyl groups, indolyl groups, carbazolyl groups, pyridyl groups, benzoquinolyl groups, fluoranthenyl groups, acenaphthofluoranthenyl groups, and stilbene groups, or preferably groups represented by the following general formulae (C) and (D).
In the general formula (C), r is an integer in a range of 1 to 3.

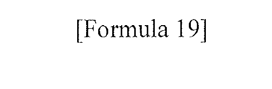

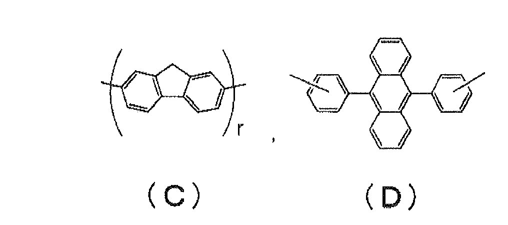

The aryl group having ring atoms of 5 to 40 may be substituted by a substituent. Preferable examples of the substituent are: an alkyl group having 1 to 6 carbon atoms such as an ethyl group, a methyl group, an isopropyl group, an n-propyl group, an s-butyl group, a t-butyl group, a pentyl group, a hexyl group, a cyclopentyl group, or a cyclohexyl group; an alkoxy group having 1 to 6 carbon atoms such as an ethoxy group, a methoxy group, an isopropoxy group, an n-propoxy group, an s-butoxy group, a t-butoxy group, a pentoxy group, a hexyloxy group, a cyclopentoxy group, or a cyclohexyloxy group; an aryl group having 5 to 40 ring atoms; an amino group substituted by an aryl group having 5 to 40 ring atoms; an ester group containing an aryl group having 5 to 40 ring atoms; an ester group containing an alkyl group having 1 to 6 carbon atoms; a cyano group; a nitro group; and a halogen atom such as chlorine, bromine, or iodine.

### (Organic Electroluminescence Device)

Next, an organic electroluminescence device will be described.

### (Arrangement of Organic Electroluminescence Device)

### (1) Arrangement of Organic Electroluminescence Device

Typical arrangement of the organic electroluminescence device may be exemplified by the following arrangements.
(a) anode/emitting layer/cathode
(b) anode/hole injecting layer/emitting layer/cathode
(c) anode/emitting layer/electron injecting layer/cathode
(d) anode/hole injecting layer/emitting layer/electron injecting layer/cathode
(e) anode/organc semiconductor layer/emitting layer/cathode
(f) anode/organic semiconductor layer/electron blocking layer/emitting layer/cathode
(g) anode/organic semiconductor layer/emitting layer/adhesion improving layer/cathode
(h) anode/hole injecting layer/hole transporting layer/emitting layer/electron injecting layer/cathode
(i) anode/insulating layer/emitting layer/insulating layer/cathode
(j) anode/inorganic semiconductor layer/insulating layer/emitting layer/insulating layer/cathode
(k) anode/organic semiconductor layer/insulating layer/emitting layer/insulating layer/cathode
(l) anode/insulating layer/hole injecting layer/hole transporting layer/emitting layer/insulating layer/cathode
(m) anode/insulating layer/hole injecting layer/hole transporting layer/emitting layer/electron injecting layer/cathode
Among these, the arrangement (h) is usually preferable.

### (2) Light-Transmissive Substrate

The organic electroluminescence device is formed on a light-transmissive substrate. The light-transmissive plate, which supports the organic electroluminescence device, is preferably a smoothly-shaped substrate that transmits 50% or more of light in a visible region of 400nm to 700nm.
The light-transmissive plate is exemplarily a glass plate, a polymer plate or the like.
For the glass plate, such materials as soda-lime glass, barium/strontium-containing glass, lead glass, aluminosilicate glass, borosilicate glass, barium borosilicate glass, quartz and the like can be used.
For the polymer plate, such materials as polycarbonate, acryl, polyethylene terephthalate, polyether sulfide, polysulfone and the like can be used.

### (3) Anode

The anode of the organic electroluminescence device is used for injecting a hole into the hole transporting layer or the emitting layer. It is effective that the anode includes a work function of 4.5 eV or more. Exemplary materials for the anode are indium-tin oxide (ITO), tin oxide (NESA), indium zinc oxide (IZO), gold, silver, platinum and copper. In order to inject electron into the electron transporting layer or the emitting layer, materials with smaller work function is more preferably used for the anode.
The anode may be made by forming a thin film from these electrode materials through methods such as vapor deposition and sputtering.
When luminescence from the emitting layer is provided through the anode, the anode preferably transmits more than 10% of the luminescence. Sheet resistance of the anode is preferably several hundreds Ω/ square or lower. Although depending on the material of the anode, thickness of the anode is typically in a range from 10 nm to 1 µm, and preferably in a range from 10 to 200 nm.

### (4) Emitting Layer

The emitting layer of the organic electroluminescence device performs functions as follows.
The luminescent layer specifically performs: an injection for allowing the hole to be injected thereinto from the anode or the hole injecting layer and allowing the electron to be injected thereinto from the cathode or the electron injecting layer when electric field is impressed; a transport function for transporting injected charge (the electron and the hole) by a force of electric field; and luminescence function for providing conditions for recombination of the electron and the hole to generate luminescence.
Although there may be a difference in degrees of easiness of receiving the injected hole and that of the injected electron and a difference in transporting capabilities represented by mobilities of the hole and the electron, the emitting layer preferably transports one of the electric charges.
As a method to form the luminescent layer, known methods such as a spin coating and an LB method may be employed.
The emitting layer is preferably a molecular deposit film.
The molecular deposit film means a thin film formed by depositing a material compound in gas phase or a film formed by solidifying a material compound in a solution state or in liquid phase. The molecular deposit film is generally distinguished from a thin film formed by the LB method (molecular accumulation film) by differences in aggregation structures, higher order structures and functional differences arising therefrom.
As disclosed in JP-A-57-51781, the emitting layer can be formed by preparing a solution by dissolving a binder (e.g. a resin) and the material compound in a solvent and forming a thin film from the solution by spin coating or the like.
The thickness of the emitting layer is preferably in the range from 5 to 50 nm, more preferably in the range from 7 to 50 nm and most preferably in the range 10 to 50 nm. The thickness below 5 nm may cause difficulty in forming the emitting layer and in controlling chromaticity, while the thickness above 50 nm may raise driving voltage.

### (5) Hole Injecting/Transporting Layers (Hole Transporting Zone)

The hole injecting/transporting layer helps injection of the hole into the emitting layer and transport the hole to a luminescent region, in which the hole mobility is large and the energy of ionization is typically small (5.5 eV or smaller). A material of the hole injecting/transporting layer is preferably such a material as to transport the hole to the emitting layer with a low field intensity, and more preferably such a material as to transport the hole with the hole mobility of at least 10⁻⁴ cm²/V sec when the exemplary electrical field of 10⁴ to 10⁶ V/cm is applied.

Examples of the material are a triazole derivative (see, for instance, the specification of US Patent No. 3,112,197), an oxadiazole derivative (see, for instance, the specification of US Patent No. 3,189,447), an imidazole derivative (see, for instance, the publication of JP-B-37-16096), a polyarylalkane derivative (see, for instance, the specifications of US Patent No. 3,615,402, No.3,820,989 and No. 3,542,544 and the publications of JP-B-45-555, JP-B-51-10983, JP-A-51-93224, JP-A-55-17105, JP-A-56-4148, JP-A-55-108667, JP-A-55-156953, and JP-A-56-36656), a pyrazoline derivative and a pyrazolone derivative (see, for instance, the specifications of US Patent No. 3,180,729 and No. 4,278,746 and the publications of JP-A-55-88064, JP-A-55-88065, JP-49-105537, JP-A-55-51086, JP-A-56-80051, JP-A-56-88141, JP-A-57-45545, JP-A-54-112637 and JP-A-55-74546, a phenylenediamine derivative (see, for instance, the specification of US Patent No. 3,615,404 and the publications of JP-B-51-10105, JP-B-46-3712, JP-B-47-25336 and JP-A-54-119925), an arylamine derivative (see, for instance, the specifications of US Patent No. 3,567,450, No. 3,240,597, No. 3,658,520, No. 4,232,103, No. 4,175,961 and No. 4,012,376 and the publications of JP-B-49-35702, JP-B-39-27577, JP-A-55-144250, JP-A-56-119132 and JP-A-56-22437 and the specification of West Germany Patent No. 1,110,518), an amino-substituted chalcone derivative (see, for instance, the specification of US Patent No. 3,526,501), an oxazole derivative (disclosed in, for instance, the specification of US Patent No. 3, 257,203), a styrylanthracene derivative (see, for instance, the publication of JP-A-56-46234), a fluorenone derivative (see, for instance, the publication of JP-A-54-110837), a hydrazone derivative (see, for instance, the specification of US Patent No. 3,717,462 and the publications of JP-A-54-59143, JP-A-55-52063, JP-A-55-52064, JP-A-55-46760, JP-A-57-11350, JP-A-57-148749 and JP-A-2-311591), a stilbene derivative (see, for instance, the publications of JP-A-61-210363, JP-A-61-228451, JP-A-61-14642, JP-A-61-72255, JP-A-62-47646, JP-A-62-36674, JP-A-62-10652, JP-A-62-30255, JP-A-60-93455, JP-A-60-94462, JP-A-60-174749 and JP-A-60-175052), a silazane derivative (see the specification of US Patent No. 4,950,950), a polysilane type (see the publication of JP-A-2-204996), an aniline-based copolymer (see the publication of JP-A-02-282263), and a conductive high-molecular oligomer (particularly, thiophene oligomer).

Although the substances listed above can be used as the material of the hole injecting/transporting layer, it is possible to use a porphyrin compound (disclosed in, for instance, the publication of JP-A-63-295695), an aromatic tertiary amine compound and a styrylamine compound (see, for instance, the specification of US Patent No. 4,127,412 and the publications of JP-A-53-27033, JP-A-54-58445, JP-A-55-79450, JP-A-55-144250, JP-A-56-119132, JP-A-61-29558, JP-A-61-98353 and JP-A-63-295695). Among these, use of the aromatic tertiary amine compound is particularly preferable.
Further examples of the material are 4,4'-bis(N-(1-naphthyl)-N-phenylamino)biphenyl (hereinafter, abbreviated as NPD) having in the molecule such two fused aromatic rings as disclosed in US Patent No. 5,061,569, 4,4',4"-tris(N-(3-methylphenyl)-N-phenylamino)triphenylamine (hereinafter, abbreviated as MTDATA) in which such three triphenylamine units as disclosed in the publication of JP-A-04-308688 are linked in a starbust form, and the like.

In addition, inorganic compounds such as p-type Si and p-type SiC can be used as the material of the hole injecting layer.

The hole injecting/transporting layer can be formed by forming thin films from the compounds listed above by known methods such as vacuum deposition, spin coating, casting and the LB method.
Although the thickness of the hole injecting/transporting layer is not particularly subject to any limitations, the thickness is typically in the range from 5 nm to 5 µm.

### (6) Electron Injecting/Transporting Layers (Electron Transporting Zone)

The electron injecting/transporting layer may further be laminated between the organic emitting layer and the cathode. The electron injecting/transporting layer, which helps injection of the electron into the emitting layer, has a high electron mobility.
It is known that, in the organic EL, since light emitted by the organic EL is reflected by an electrode (the cathode, in this case), light directly taken out from the anode and the light taken out after being reflected by the electrode interfere with each other. In order to efficiently utilize the interference, the thickness of the electron transporting layer is suitably selected from the range of several nanometers to several micrometers. However, especially when the thickness of the layer is large, the electron mobility is preferably at least 10⁻⁵ cm²/Vs or higher so as to prevent voltage rise when the electrical field of 10⁴ to 10⁶ V/cm is applied.
As a material used for the electron inj ecting/transporting layer, 8-hydroxyquinoline or a metal complex of its derivative is preferable. Examples of the 8-hydroxyquinoline or the metal complex of its derivative are metal chelate oxynoid compounds containing a chelate of oxine (typically 8-quinolinol or 8-hydroxyquinoline). For example, Alq having Al as its central metal can be used for the electron injecting/transporting layer.

An oxadiazole derivative represented by the formula below is also preferable as a material of the electron injecting (transporting) layer.

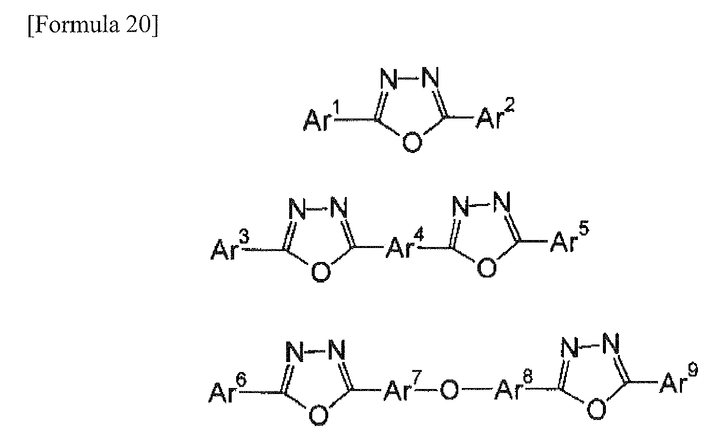

(In the formula, Ar¹,Ar²,Ar³,Ar⁵,Ar⁶ and Ar⁹ each represent a substituted or unsubstituted aryl group, which may be the same or different from one another. Ar⁴,Ar⁷ and Ar⁸ each represent a substituted or unsubstituted arylene group, which may be the same or different from one another.)

Examples of the aryl group are a phenyl group, a biphenyl group, an anthranil group, a chrysenyl group, a perylenyl group, and a pyrenyl group.
Examples of the arylene group are a phenylene group, a naphtylene group, a biphenylene group, an anthranylene group, a chrysenylene group, a perylenylene group and a pyrenylene group. The substituent group may include an alkyl group having 1 to 10 carbon atoms, an alkoxy group having 1 to 10 carbon atoms and a cyano group. The electron transporting compounds are preferably compounds that exhibit favorable performance in forming a thin film.
Examples of the electron transporting compounds are as follows.

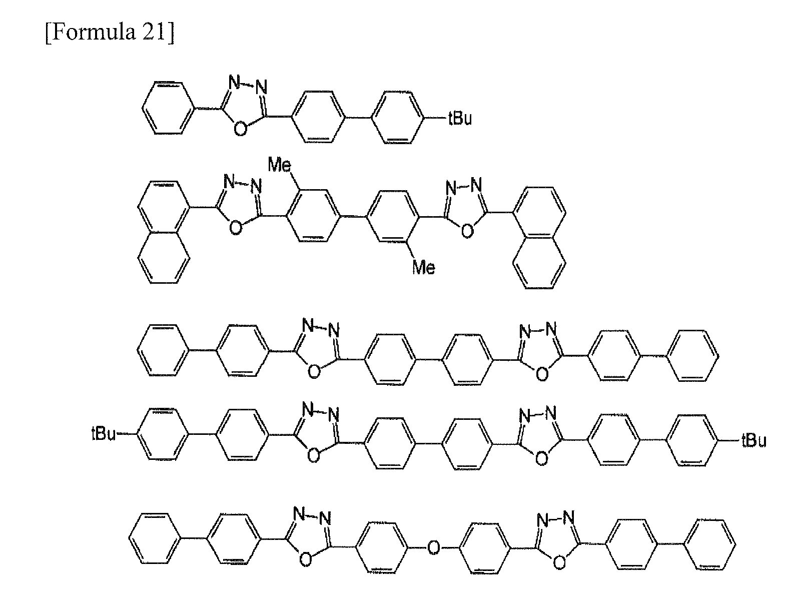

A nitrogen-containing heterocycle derivative represented by the formula below is also preferable as a material of the electron injecting (transporting) layer.

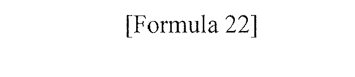

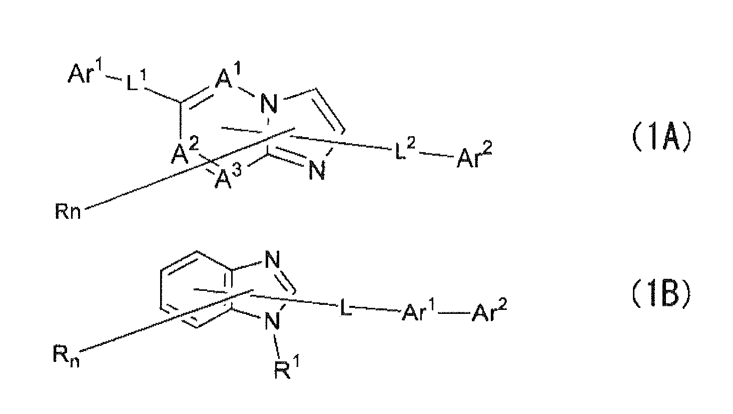

In the formula, A¹ to A³ each represent a nitrogen atom or a carbon atom; R represents a substituted or unsubstituted aryl group having 6 to 60 carbon atoms, a substituted or unsubstituted heteroaryl group having 3 to 60 carbon atoms, an alkyl group having 1 to 20 carbon atoms, a haloalkyl group having 1 to 20 carbon atoms or an alkoxy group having 1 to 20 carbon atoms; and n represents an integer in a range of 0 to 5, where the plurality of R may be the same or different from one another when n is an integer of 2 or larger.
In addition, a plurality of adjacent Rs may be bonded to each other to form a substituted or unsubstituted carbocyclic aliphatic ring or a substituted or unsubstituted carbocyclic aromatic ring.
Ar¹ represents a substituted or unsubstituted aryl group having 6 to 60 carbon atoms or a substituted or unsubstituted heteroaryl group having 3 to 60 carbon atoms; and Ar² represents a hydrogen atom, an alkyl group having 1 to 20 carbon atoms, a haloalkyl group having 1 to 20 carbon atoms, an alkoxy group having 1 to 20 carbon atoms, a substituted or unsubstituted aryl group having 6 to 60 carbon atoms or a substituted or unsubstituted heteroaryl group having 3 to 60 carbon atoms, one of Ar¹ and Ar² being a substituted or unsubstituted fused ring group having 10 to 60 carbon atoms or a substituted or unsubstituted fused heterocyclic group having 3 to 60 carbon atoms. L¹ and L² each represent a single bond, a substituted or unsubstituted fused ring having 6 to 60 carbon atoms, a substituted or unsubstituted fused heterocycle having 3 to 60 carbon atoms or a substituted or unsubstituted fluorenylene group.

[Formula 23] HAr-L¹-Ar¹-Ar²

In the formula, HAr represents a substituted or unsubstituted nitrogen-containing ring having 3 to 40 carbon atoms; L¹ represents a single bond, a substituted or unsubstituted arylene group having 6 to 60 carbon atoms, a substituted or unsubstituted heteroarylene group having 3 to 60 carbon atoms or a substituted or unsubstituted fluorenylene group; Ar¹ represents a substituted or unsubstituted divalent aromatic hydrocarbon group having 6 to 60 carbon atoms; and Ar² represents a substituted or unsubstituted aryl group having 6 to 60 carbon atoms, or a substituted or unsubstituted heteroaryl group having 3 to 60 carbon atoms.

A silacyclopentadiene derivative represented by the formula below is also preferable as a material of the electron injecting (transporting) layer.

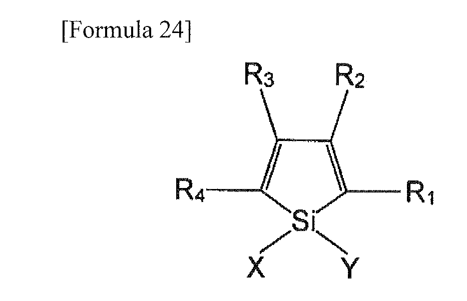

In the formula, X and Y may each represent a saturated or unsaturated hydrocarbon group having 1 to 6 carbon atoms, an alkoxy group, an alkenyloxy group, an alkynyloxy group, a hydroxy group, a substituted or unsubstituted aryl group, a substituted or unsubstituted heterocycle or X and Y may be bonded to form a saturated or unsaturated ring. R₁ to R₄ may each represent hydrogen, halogen, a substituted or unsubstituted alkyl group having 1 to 6 carbon atoms, an alkoxy group, an aryloxy group, a perfluoroalkyl group, a perfluoroalkoxy group, an amino group, an alkylcarbonyl group, an arylcarbonyl group, an alkoxycarbonyl group, an aryloxycarbonyl group, an azo group, an alkylcarbonyloxy group, an arylcarbonyloxy group, an alkoxycarbonyloxy group, an aryloxycarbonyloxy group, a sulfinyl group, a sulfonyl group, a sulfanyl group, a silyl group, a carbamoyl group, an aryl group, a heterocyclic group, an alkenyl group, an alkynyl group, a nitro group, a formyl group, a nitroso group, a formyloxy group, an isocyano group, a cyanate group, an isocyanate group, a thiocyanate group, an isothiocyanate group or cyano group, or an adjacent set of R₁ to R₄ may be fused to form a substituted or unsubstituted ring.

A borane derivative represented by the formula below is also preferable as a material of the electron injecting (transporting) layer.

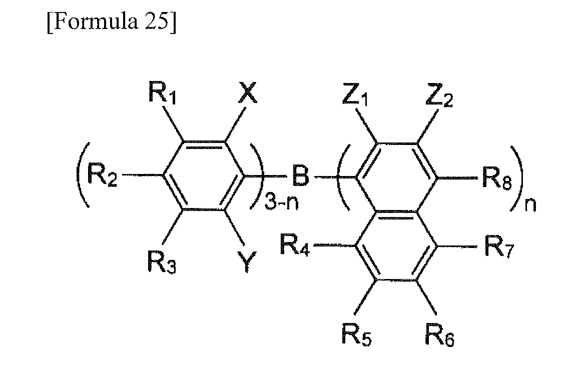

(In the formula, R1 to R4 and Z2 are each represent a hydrogen atom, a saturated or unsaturated hydrocarbon group, an aromatic group, a heterocyclo group, a substituted amino group, a substituted boryl group, an alkoxy group or an aryloxy group; X, Y and Z1 are each represent a saturated or unsaturated hydrocarbon group, an aromatic group, a heterocyclo group, a substituted amino group, an alkoxy group or an aryloxy group; substituent groups of Z1 and Z2 may be bonded to form a fused ring; and n represents an integer of 1 to 3, where when n is equal to or larger than two, Z may be different.
However a condition in which n is 1, X, Y and R₂ are the methyl group and R₈ is the hydrogen atom or the substituted boryl group and a condition in which n is 3 and Z₁ is the methyl group are excluded.

A gallium complex represented by the formula below is also preferable as a material of the electron injecting (transporting) layer.

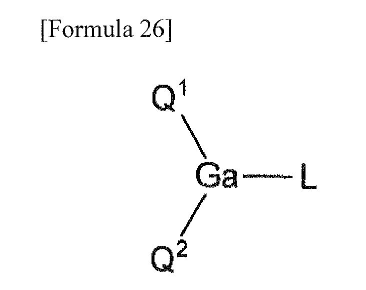

In this formula, Q¹ and Q² each represent a ligand shown by the formula below. L represents a ligand which may be a halogen atom; a substituted or unsubstituted alkyl group; a substituted or unsubstituted cycloalkyl group; a substituted or unsubstituted aryl group; a substituted or unsubstituted heterocyclic group; those represented by -OR¹ (R¹ representing a hydrogen atom, a substituted or unsubstituted alkyl group, a substituted or unsubstituted cycloalkyl group, a substituted or unsubstituted aryl group or a substituted or unsubstituted heterocyclic group); or those represented by -O-Ga-Q³(Q⁴) (Q³ and Q⁴ being the same as Q¹ and Q²).

In the formula, Q1 to Q4 each represent a residue represented by the formula below, which may be exemplified by, but not limited to, a quinoline residue such as 8-hydroxyquinoline and 2-methyl-8-hydroxyquinoline.

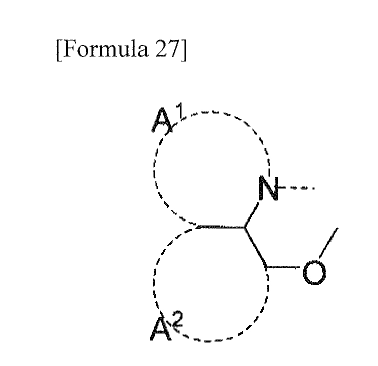

Rings A¹ and A² are bonded to each other, Rings A¹ and A² being substituted or unsubstituted aryl rings bonded to each other or a heterocyclic structure.

The metal complex shown above exhibits a strong property as an n-type semiconductor and has a large electron injecting capability. In addition, formation energy required when forming the complex is low, so that bonding between the metal and the ligand in the formed metal complex becomes strong, thus exhibiting a large fluorescence quantum efficiency as a luminescent material.

Examples of the substituent groups of Ring A¹ and Ring A² that form the ligands in the formula above are: halogen atoms such as chlorine, bromine, iodine and fluorine; substituted or unsubstituted alkyl groups such as a methyl group, an ethyl group, a propyl group, a butyl group, a sec-butyl group, a tert-butyl group, a pentyl group, a hexyl group, a heptyl group, an octyl group, a stearyl group and a trichloromethyl group; substituted or unsubstituted aryl groups such as a phenyl group, a naphthyl group a 3-methylphenyl group, a 3-methoxyphenyl group, a 3-fluorophenyl group, a 3-trichloromethylphenyl group, a 3-trifluoromethylphenyl group and a 3-nitrophenyl group; substituted or unsubstituted alkoxy groups such as a methoxy group, a n-butoxy group, a tert-butoxy group, a trichloromethoxy group, a trifluoroethoxy group,a pentafluoropropoxy group, a 2,2,3,3-tetrafluoropropoxy group, a 1,1,1,3,3,3-hexafluoro-2-propoxy group and a 6-(perfluorohethyl)hexyloxy group; substituted or unsubstituted aryloxy groups such as a phenoxy group, a p-nitrophenoxy group, a p-tert-butylphenoxy group, a 3-fluorophenoxy group, a pentafluorophenyl group and a 3-trifluoromethylphenoxy group; substituted or unsubstituted alkylthio groups such as a methylthio group, an ethylthio group, a tert-butylthio group, a hexylthio group, an octylthio group and a trifluoromethylthio group; substituted or unsubstituted arylthio groups such as a phenylthio group, a p-nitrophenylthio group, a p-tert-butylphenylthio group, a 3-fluorophenylthio group, a pentafluorophenylthio group and a 3-trifluoromethylphenylthio group; mono- or disubstituted amino groups such as a cyano group, a nitro group, an amino group, a methylamino group, a diethylamino group, an ethylamino group, a diethylamino group, a dipropylamino group, a dibutylamino group and a diphenylamino group; acylamino groups such as a bis(acetoxymethyl) amino group, a bis(acetoxyethyl) amino group, a bis (acetoxypropyl) amino group and a bis(acetoxybutyl) amino group; a hydroxyl group; a siloxy group; an acyl group; carbamoyl groups such as a methylcarbamoyl group, a dimethylcarbamoyl group, an ethylcarbamoyl group, a diethylcarbamoyl group, a propylcarbamoyl group, a butylcarbamoyl group, and a phenylcarbamoyl group; a carboxylic acid group; a sulfonic acid group; an imide group; cycloalkyl groups such as a cyclopentane group and a cyclohexyl group; aryl groups such as a phenyl group, a naphthyl group, a biphenyl group, an anthranil group, a phenanthryl group, a fluorenyl group and a pyrenyl group; and heterocyclic groups such as a pyridinyl group a pyrazinyl group, a pyrimidinyl group, a pyridazinyl group, a triazinyl group, an indolinyl group, a quinolinyl group, an acridinyl group, a pyrrolidinyl group, a dioxanyl group, a piperidinyl group, a morpholidinyl group, a piperazinyl group, a triathinyl group, a carbazolyl group, a furanyl group, a thiophenyl group, an oxazolyl group, an oxadiazolyl group, a benzoxazolyl group, a thiazolyl group, a thiadiazolyl group, a benzothiazolyl group, a triazolyl group, an imidazolyl group and a benzoimidazolyl group. In addition, the substituent groups listed above may be bonded to each other to form a 6-membered aryl ring or a heterocycle.

As a preferred embodiment of the organic EL device, there is known a device containing a reductive dopant at a boundary between a region transporting the electron or the cathode and an organic layer. Here, the reductive dopant is defined as a substance capable of reducing an electron transporting compound. Thus, various substances having a certain level of reducibility can be used, preferable examples of which are at least one substance selected from a group consisting of: alkali metal, alkali earth metal, rare earth metal, an oxide of the alkali metal, a halogenide of the alkali metal, an oxide of the alkali earth metal, a halogenide of the alkali earth metal, an oxide of the rare earth metal, a halogenide of the rare earth metal, an organic complex of the alkali metal, an organic complex of the alkali earth metal and an organic complex of the rare earth metal.

Specifically, reductive dopant is preferably a substance(s) having the work function of 2.9 eV or lower, which is exemplified by at least one alkali metal selected from a group consisting of Li (work function: 2.9 eV), Na (work function: 2.36 eV), K (work function: 2.28 eV), Rb (work function: 2.16 eV) and Cs (work function: 1.95 eV) or at least one alkali earth metal selected from a group consisting of Ca (work function: 2.9 eV), Sr (work function: 2.0 to 2.5 eV) and Ba (work function: 2.52 eV), and the substances having the work function of 2.9 eV or lower are particularly preferable. Among these, more preferable reductive dopant is at least one alkali metal selected from a group consisting of K, Rb and Cs, in which Rb and Cs are even more preferable and Cs is the most preferable. These alkali metals have particularly high reducibility, so that addition of a relatively small amount of these alkali metals to an electron injection region can enhance luminescence intensity and lifecycle of the organic EL device. In addition, as the reductive dopant having the work function of 2.9 eV or lower, a combination of two or more of these alkali metals is also preferable, and a combination including Cs is particularly preferable (e.g. combinations of Cs an Na, Cs and K, Cs and Rb or Cs, Na and K). The combinations including Cs can effectively exert the reducibility, so that the addition of such reductive dopant to the electron injecting zone can enhance the luminescence intensity and the lifecycle of the organic electroluminescence device.

An electron injecting layer formed from an insulator or a semiconductor may be provided between the cathode and the organic layer. With the arrangement, leak of electric current can be effectively prevented and the electron injecting capability can be enhanced. As the insulator, it is preferable to use at least one metal compound selected from a group consisting of an alkali metal chalcogenide, an alkali earth metal chalcogenide, a halogenide of alkali metal and a halogenide of alkali earth metal. By forming the electron injecting layer from the alkali metal chalcogenide or the like, the electron injecting capability can preferably be further enhanced. Specifically, preferable examples of the alkali metal chalcogenide are Li2O, K2O, Na2S, Na2Se and Na2O, while preferable example of the alkali earth metal chalcogenide are CaO, BaO, SrO, BeO, BaS and CaSe. Preferable examples of the halogenide of the alkali metal are LiF, NaF, KF, LiCl, KCl and NaCl. Preferable examples of the halogenide of the alkali earth metal are fluorides such as CaF2, BaF2, SrF2, MgF2 and BeF2, and halogenides other than the fluoride.
Examples of the semiconductor for forming the electron transporting layer are one of or a combination of two or more of an oxide, a nitride or an oxidized nitride containing at least one element selected from a group consisting of Ba, Ca, Sr, Yb, Al, Ga, In, Li, Na, Cd, Mg, Si, Ta, Sb and Zn. An inorganic compound for forming the electron transporting layer is preferably a microcrystalline or amorphous semiconductor film. When the electron transporting layer is formed of such semiconductor film, more uniform thin film can be formed, thereby reducing pixel defects such as a dark spot. Examples of such an inorganic compound are the above-described alkali metal chalcogenide, alkali earth metal chalcogenide, halogenide of the alkali metal and halogenide of the alkali earth metal.

### (7) Cathode

In order to inject the electron into the electron injecting/transporting layer or the emitting layer, a material whose work function is small (4 eV or lower) is used as an electrode material for the cathode, examples of the material being metals, alloys, electrically conductive compounds and mixtures thereof. Examples of the electrode material are sodium, a sodium-potassium alloy, magnesium, lithium, a magnesium-silver alloy, aluminium/aluminium oxide, an aluminium-lithium alloy, indium, rare earth metal and the like.
The cathode may be made by forming a thin film from the electrode material by vapor deposition and sputtering.
When luminescence from the emitting layer is provided through the cathode, the cathode preferably transmits more than 10% of the luminescence.
The sheet resistance as the cathode is preferably several hundreds Ω/ square or lower, and the thickness of the film is typically in a range from 10 nm to 1 µm, preferably 50 to 200 nm.

### (8) Insulating Layer

Since the electrical field is applied to ultra thin films in the organic electroluminescence device, pixel defects resulted from leak or short circuit likely occur. In order to prevent such defects, it is preferable to interpose an insulating thin film layer between a pair of electrodes.
Examples of a material used for the insulating layer are aluminum oxide, lithium fluoride, lithium oxide, cesium fluoride, cesium oxide, magnesium oxide, magnesium fluoride, calcium oxide, calcium fluoride, aluminium nitride, titanium oxide, silicon oxide, germanium oxide, silicon nitride, boron nitride, molybdenum oxide, ruthenium oxide, vanadium oxide and the like.
Mixtures or laminates thereof may also be used.

### (9) Manufacturing Method of Organic Electroluminescence Device

The organic electroluminescence device can be manufactured by forming the anode, the emitting layer, the hole injecting layer (as necessary), the electron injecting layer (as necessary) and the cathode form the materials listed above by the above-described formation methods. The organic electroluminescence device can be manufactured by forming the above elements in the inverse order of the above, namely from the cathode to the anode.

The following is an example of a manufacturing method of the organic electroluminescence device in which the anode, the hole injecting layer, the emitting layer, the electron injecting layer and the cathode are sequentially formed on the light-transmissive substrate.

A thin film is formed of the anode material on a suitable light-transmissive substrate by vapor deposition or sputtering such that the thickness of the thin film is 1 µm or smaller, preferably in a range from 10 nm to 200 nm, thereby forming the anode.
Then, the hole injecting layer is formed on the formed anode.
The hole injecting layer can be formed by vacuum deposition, spin coating, casting method, LB method or the like. The thickness of the hole injecting layer is suitably determined within a range of 5 nm to 5 µm.

Then, the emitting layer is formed on the hole injecting layer by forming a thin film from an organic luminescent material by a dry process represented by the vacuum deposition or a wet process such as spin coating and casting method. In light of size increase in screen, reduction of cost and simplification of manufacturing process, the wet process is more preferable.

Then, the electron injecting layer is formed on the emitting layer.
The electron injecting layer may be exemplarily formed by vacuum deposition.

Lastly, the cathode is laminated on the electron injecting layer, whereby the organic EL device can be obtained.
The cathode can be formed from a metal by a method such as vapor deposition and sputtering.
In order to protect the organic layers deposited under the cathode from being damaged, the vacuum deposition is preferable.

The methods for forming each of the layers in the organic electroluminescence device are not particularly limited.
Conventional methods such as vacuum deposition and spin coating can be employed for forming the organic film layers. Specifically, the organic film layers may be formed by a conventional coating method such as vacuum deposition, molecular beam epitaxy (MBE method) and coating methods using a solution such as a dipping, spin coating, casting, bar coating, roll coating and ink jet printing.
Although the thickness of each organic layer of the organic electroluminescence device is not particularly limited, the thickness is generally preferably in a range of several nanometers to 1 µm, since excessively-thinned film likely entails defects such as a pin hole while excessively-thickened film requires high voltage to be applied and deteriorates efficiency.
When a direct current is applied to the organic electroluminescence device, the luminescence can be observed by applying a voltage of 5 to 40V with the anode having the positive polarity and the cathode having the negative polarity. When the voltage is applied with the inversed polarity, no current flows, so that the luminescence is not generated. When an alternating current is applied, the uniform luminescence can be observed only when the anode has the positive polarity and the cathode has the negative polarity. A waveform of the alternating current to be applied may be suitably selected.

### (Manufacturing Example of Organic Electroluminescence Device)

Next, manufacturing examples of the organic electroluminescence device will be described below.
A glass substrate (size: 25 mm x 75 mm x 1.1 mm thick) having an ITO transparent electrode (manufactured by Geomatics) is ultrasonic-cleaned in isopropyl alcohol for five minutes, and then UV/ozone-cleaned for 30 minutes.
Polyethylene-dioxy-thiophene / polystyrene sulphonic acid (PEDOT/PSS) for forming the hole injecting layer was deposited on the substrate by spin coating to form a film of 100 nm thick.
A film of 20 nm thick was formed from a toluene solution (0.6 wt%) of below-described Polymer 1 (Mw: 145000) by spin coating and dried at 170°C for 30 minutes.
The emitting layer was subsequently formed in a film form by spin coating from a toluene solution containing the compound AN-1 and a compound BD-1 by 1 wt% with a ratio of AN-1 to BD-1 being 20 to 1 (wt/wt).
The thickness was 50 nm at this time.
Then, a tris(8-quinolinol)aluminum film (hereinafter, abbreviated as Alq film) of 10 nm thick was formed on the emitting layer.
The Alq film serves as the electron transporting layer.
Li (Li source: manufactured by SAES Getters Corporation) as the reductive dopant and Alq were co-deposited to form an Alq:Li film as the electron injecting layer (cathode).
Metal (Al) was vapor-deposited on the Alq:Li film to form a metal cathode, such that the organic electroluminescence device was provided.
The device emitted blue light and its light-emission surface was uniform.
The luminescence efficiency at this time was 5.2 cd/A, and time elapsed until the luminescent intensity decreased to half was 1500 hours with the initial luminescence intensity being 1000 ed/m²

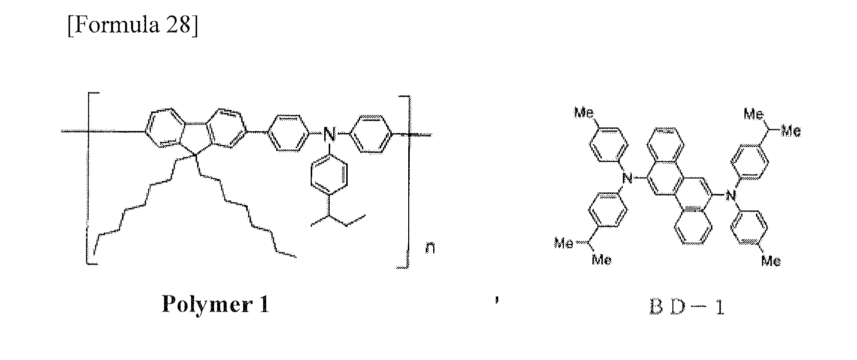

### (Comparatives of Organic Electroluminescence Device)

Organic electroluminescence devices were manufactured as was the organic electroluminescence device according to the above manufacturing example 1.
However, a below-described compound (a) was used in place of AN-1.
The compound (a) was prepared by adding substituent(s) to the central anthracene skeleton.
The compound (a), whose solubility was evaluated in advance, exhibited high solubility of 10 wt% in toluene.
The device prepared in the above manner emitted blue light and its light-emission surface was uniform.
However, the luminescence efficiency was as low as 4.1 cd/A, and time elapsed until the luminescent intensity decreased to half was 460 hours with the initial luminescence intensity being 1000 cd/m².

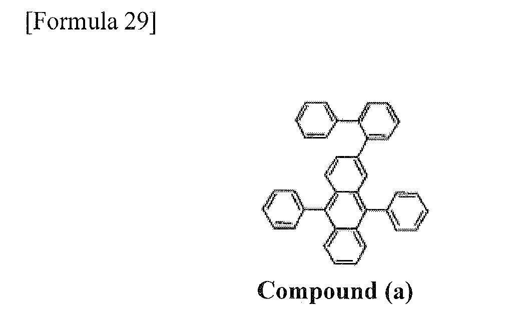

As is understood from the comparative, the solubility of the compound can also be improved by introducing aryl substituent at 2-position of anthracene
However, the introduction of substituent at 2-position of central anthracene skeleton deteriorates performance of the device to such a degree as to make the device practically not applicable.
Specifically, in order to obtain a compound that is excellent both in solubility and device performance, it has been proved necessary to identify at which position(s) structure(s) for enhancing solubility (soluble portion B) should be bonded in anthracene (basic skeleton).

The present invention is not limited to the above-described embodiment(s) or example(s).
For instance, the structures and the bonding positions of Ar₂ and Ar₃ in the formulae (3) and (4) respectively subject to no specific limitation.
Examples of Ar₂ and Ar₃ other than a phenyl group are fused rings such as a naphthyl group or a terphenyl group and derivatives thereof.
Further, instead of being a fused ring or the like, Ar₂ and Ar₃ may be an aromatic hydrocarbon group that is exemplarily structured as follows.
Specifically, the present invention may include a structure where: the soluble portion B is bonded to the organic electroluminescence-functional portion A; the number of the units having phenyl groups (either one of the structures represented by the formula (3) and the structure(s) represented by the formula (4)) in the soluble portion B is in a range of 3 or more and 20 or less; and the number (a) of the bonds in ortho positions and meta positions in the phenyl groups of the units having the phenyl groups is larger than the number (b) of the bonds in para positions therein.

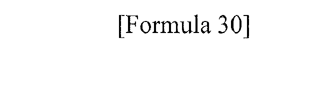

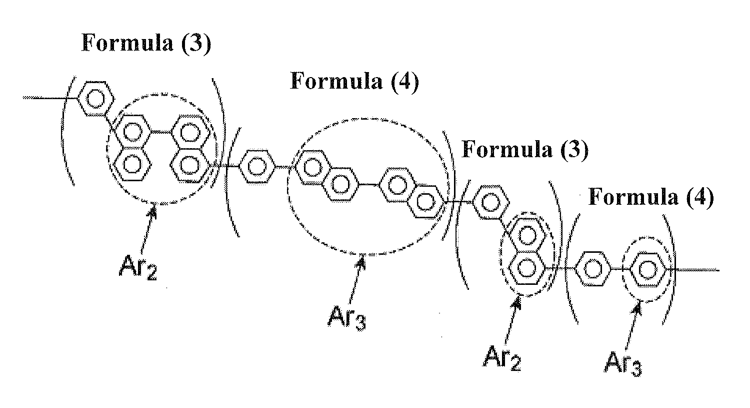

### INDUSTRIAL APPLICABILITY

This invention can be used in manufacturing an organic electroluminescent display.

## Claims

1. An organic electroluminescent material-containing solution, comprising:
an organic electroluminescent material; and
a solvent, wherein
the organic electroluminescent material contains a host and a dopant,
the host is a compound comprising: an organic electroluminescence-functional portion A formed of a low-molecular organic electroluminescent material; and a soluble portion B bonded to the organic electroluminescence-functional portion A for solubilizing the low-molecular organic electroluminescent material in the solvent, the compound being represented by following formula (1),
the organic electroluminescence-functional portion A in the formula (1) is represented by following formula (2),
the soluble portion B in the formula (1) is formed by bonding either plural structures represented by following formula (3) or plural structures represented by following formula (4) or by bonding plural structures represented by the formula (3) with structure(s) represented by the formula (4), and
a number (a) and a number (b) satisfies both relationships respectively represented by 3 ≤ (a) + (b) ≤ 20 and (a) ≥ (b), the number (a) being the number of the structures represented by the formula (3), the number (b) being the number of the structure(s) represented by the formula (4).
[Formula 1] A-B-E (1)
(E represents a terminal group) 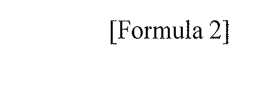 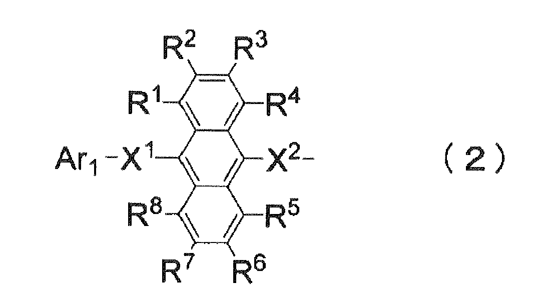 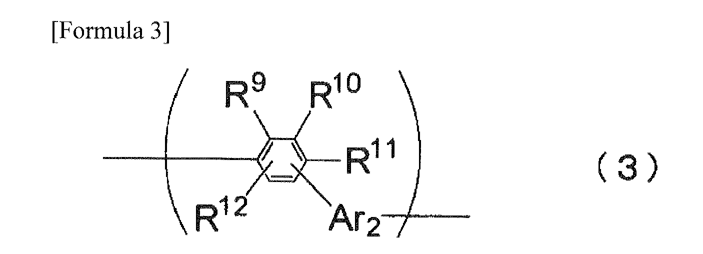 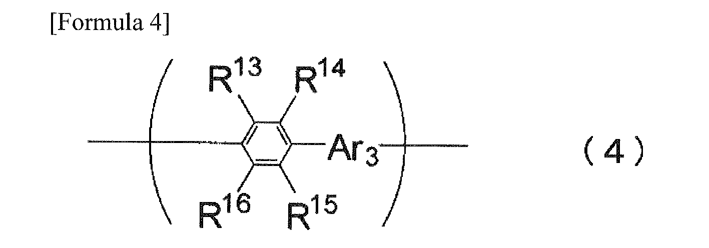 (Where: Ar₁, Ar₂ and Ar₃ each represent a substituted or unsubstituted aromatic hydrocarbon group having 6 to 50 carbon atoms;
X¹ and X² each represent a single bond or a divalent substituted or unsubstituted aromatic hydrocarbon group having 6 to 50 carbon atoms; and
R¹ to R¹⁶ each are selected from a group consisting of a hydrogen atom, a substituted or unsubstituted aromatic hydrocarbon group, a substituted or unsubstituted aromatic heterocyclic group, a substituted or unsubstituted alkyl group, a substituted or unsubstituted cycloalkyl group, a substituted or unsubstituted alkoxy group, a substituted or unsubstituted aralkyl group, a substituted or unsubstituted aryloxy group, a substituted or unsubstituted arylthio group, a substituted or unsubstituted alkoxycarbonyl group, a substituted or unsubstituted silyl group, a carboxyl group, a halogen atom, a cyano group, a nitro group and a hydroxyl group,
an adjacent set of R¹ to R¹⁶ is allowed to be mutually bonded to form a cyclic structure.)

2. The organic electroluminescent material-containing solution according to Claim 1, wherein the structures represented by the formula (4) are not located next to each other in a structure of the soluble portion B of the formula (1).

3. The organic electroluminescent material-containing solution according to Claim 1 or 2, wherein the Ar₂ in the formula (3) and the Ar₃ in the formula (4) in the soluble portion B are respectively a phenyl group.

4. The organic electroluminescent material-containing solution according to any one of Claims 1 to 3, wherein
the soluble portion B is formed by bonding the structures represented by the formula (3) with the structure(s) represented by the formula (4), and
a dispersion of a bonding number U is a single value, the bonding number U representing a sum of the number (a) of the structures represented by the formula (3) and the number (b) of the structure(s) represented by the formula (4).

5. The organic electroluminescent material-containing solution according to any one of Claims 1 to 4, wherein the soluble portion B is entirely formed of the structures represented by the formula (3).

6. The organic electroluminescent material-containing solution according to any of one Claims 1 to 5, wherein the solvent is selected from a group consisting of an aromatic solvent, a halogen-based solvent and an ether-based solvent.

7. The organic electroluminescent material-containing solution according to any one of Claims 1 to 6, wherein
the solvent is selected from a group consisting of an aromatic solvent, a halogen-based solvent and an ether-based solvent, and
the solvent is further added with a viscosity control reagent selected from a group consisting of an alcohol-based solution, a ketone-based solution, a paraffin-based solution and an alkyl-substituted aromatic solution having 4 or more carbon atoms.

8. The organic electroluminescent material-containing solution according to Claim 7, wherein
the solvent is the aromatic solvent, and
the viscosity control reagent is the alcohol-based solution or the alkyl-substituted aromatic solution having 4 or more carbon atoms.

9. A method of synthesizing an organic electroluminescent material to be dissolved in the organic electroluminescent material-containing solution according to any one of Claims 1 to 8, comprising:
synthesizing the low-molecular organic luminescent material as the organic electroluminescence-functional portion A;
synthesizing a molecular chain as the soluble portion B; and
bonding the low-molecular organic electroluminescent material synthesized in the step of synthesizing the low-molecular organic electroluminescent material with the molecular chain synthesized in the step of synthesizing the molecular chain.

10. A compound synthesized by the synthesizing method according to Claim 9.

11. A method of forming thin film(s) of an organic electroluminescent material, comprising:
dropping the organic electroluminescent material-containing solution according to any one of Claims 1 to 8 on a formation area; and
forming film(s) of the organic electroluminescent material by evaporating the solvent in the organic-electroluminescence-material-containing solution dropped in the step of dropping.

12. A thin film(s) of an organic electroluminescent material formed by the method of forming thin film(s) of an organic electroluminescent material according to Claim 11.

13. An organic electroluminescence device, comprising the thin film(s) of an organic electroluminescent material according to Claim 12.

14. The organic electroluminescence device according to Claim 13, the device further comprising
an emitting layer that comprises the thin film(s) formed from the organic electroluminescent material-containing solution by coating method, wherein
the emitting layer emits light of blue color.
